# EUROPÄISCHE PATENTSCHRIFT

(11) **EP 2 797 854 B1**
(45) Veröffentlichungstag und Bekanntmachung des Hinweises auf die Patenterteilung: **19.04.2017**
(21) Anmeldenummer: 12813378.2
(22) Anmeldetag: 28.12.2012
(51) Int. Cl.: C05C 9/00, C05D 5/00

(54) **ZUSAMMENSETZUNG EINER MAGNESIUMSULFAT-HARNSTOFF-VERBINDUNG**
COMPOSITION OF A MAGNESIUM SULPHATE UREA COMPOUND
COMPOSITION D'UNE LIAISON D'URÉE ET DE SULFATE DE MAGNÉSIUM

(30) Priorität: 30.12.2011 EP 11196218
(43) Veröffentlichungstag der Anmeldung: 05.11.2014
(73) Patentinhaber: K+S KALI GmbH, 34131 Kassel (DE)
(72) Erfinder: KLEINE-KLEFFMANN, Ulrich, 36251 Bad Hersfeld (DE); WALCZYK, Wolfgang, 36266 Heringen (DE)
(74) Vertreter: Dressel, Stefan
(86) Internationale Anmeldenummer: PCT/EP2012/077017
(87) Internationale Veröffentlichungsnummer: WO 2013/098367

(56) Entgegenhaltungen:
- DE-C1- 4 232 567
- GB-A- 1 359 884
- DATABASE CA [Online] CHEMICAL ABSTRACTS SERVICE, COLUMBUS, OHIO, US; 1. Januar 1976 (1976-01-01), SHKROBOT, G. P. ET AL: "Magnesium sulfate-cobalt sulfate-urea-water system at 20.degree.C", XP002678411, gefunden im STN Database accession no. 1976:437787 in der Anmeldung erwähnt & SHKROBOT, G. P. ET AL: "Magnesium sulfate-cobalt sulfate-urea-water system at 20.degree.C", ZHURNAL NEORGANICHESKOI KHIMII , 21(5), 1427-9 CODEN: ZNOKAQ; ISSN: 0044-457X, 1976,
- COLIN W. WHITTAKER, FRANK O. LUNSDTROM AND JAMES H. SHIMP: "The System Magnesium Sulfate-Urea-Water at 30°C", JOURNAL OF AMERICAN SOCIETY, Bd. 58, 1. Oktober 1936 (1936-10-01), Seiten 1975-1977, XP002678412, US in der Anmeldung erwähnt
- VON RHEINBABEN W: "Effect of magnesium sulphate addition to urea on nitrogen loss due to ammonia volatilization", FERTILIZER RESEARCH, KLUWER ACADEMIC PUBLISHERS, DORDRECHT, NL, Bd. 11, Nr. 2, 1. Januar 1987 (1987-01-01) , Seiten 149-159, XP008152705, ISSN: 0167-1731, DOI: 10.1007/BF01051058

## Beschreibung

Die vorliegende Erfindung betrifft Zusammensetzungen einer Magnesiumsulfat-Harnstoff-Verbindung der Formel I

[MgSO₄ • m CO(NH₂)₂ • n H₂O] (I)

worin m im Bereich von 0,9 bis 1,1 und n im Bereich von 2,9 bis 3,1 liegen, die Herstellung der Zusammensetzungen und deren Verwendung als Düngemittel oder Düngemittelzusatz.

Obwohl Magnesium als achthäufigstes Element zu etwa 1,94 % in der Erdkruste vorhanden ist, weisen Böden oft einen Mangel an Magnesium auf Deshalb finden Magnesiumsalze als Düngemittel oder Düngemittelzusätze eine breite Verwendung. Insbesondere werden Salze des Magnesiums als Düngemittel oder Düngemittelzusätze eingesetzt. Üblicherweise werden diese Salze als Magnesiumsulfat in Verbindung mit Makronährstoffen wie Kalium, Phosphor oder Stickstoff sowie mit Spurenelementen wie Mangan, Zink, Kupfer, Eisen, Kobalt, Molybdän oder Bor eingesetzt.

Grundsätzlich ist es von Interesse, möglichst viele Makro- und Mikronährstoffe gemeinsam in einer Düngemittelzusammensetzung bereitzustellen. Jedoch sind dem gemeinsamen Einsatz von Magnesium und Stickstoff Grenzen gesetzt. So ist eine einfache Feststoffmischung von Bittersalz (MgSO₄ • 7 H₂O) und Harnstoff nicht lagerstabil. Es kommt schon nach kurzer Zeit zu einer Reaktion der beiden Mischungspartner, bei der sich pastöse Massen bilden, die aufgrund ihrer hohen Hygroskopizität zudem leicht zerfließen und daher schlecht zu handhaben sind und sich insbesondere nicht in feste Düngemittelzusammensetzungen einarbeiten lassen.

Magnesiumsulfat-Harnstoff-Verbindungen der Formel I sind prinzipiell aus der Literatur bekannt. So beschreiben C. W. Whittaker et al. Journal of American Society, 1936, 58, 1975 beschreiben Untersuchungen zum Phasendiagramm von Magnesiumsulfat-Harnstoff-Wasser bei 30°C. Als eine der Phasen wird eine Verbindung der Formel MgSO₄• CO(NH₂)₂ • 3 H₂O postuliert. Zur Herstellung dieser Verbindung werden äquimolare Mengen von Bittersalz (MgSO₄ • 7 H₂O) mit Harnstoff vermengt. Hierbei scheidet sich ein kristallines Produkt ab. Eigene Untersuchungen ergaben, dass es sich hier um ein Gemisch aus der Verbindung der Formel MgSO₄ • CO(NH₂)₂ • 3 H₂O mit Bittersalz (MgSO₄ • 7 H₂O) und ungebundenem Harnstoff und nicht um die Reinsubstanz MgSO₄ • CO(NH₂)₂ • 3 H₂O handelt. Eine Gewinnung der Verbindung MgSO₄ • CO(NH₂)₂ • 3 H₂O aus diesem Gemisch ist nicht möglich, da sich bei Versuchen der Aufgreinigung die Verbindung zersetzt.

Shkrobot et al., Zhurnal Neorganicheskoi Khimii, 21(5) beschreiben im Zusammenhang mit der Untersuchung von Phasendiagrammen des quaternären Systems MgSO₄-CoSO₄-CO(NH₂)₂-H₂O auch die Bildung einer festen Phase, die MgSO₄ • CO(NH₂)₂ • 3 H₂O neben größeren Mengen an Harnstoff enthält. Es ist nicht möglich, aus diesem Gemisch MgS0₄ • CO(NH₂)₂ • 3 H₂O in reiner Form zu gewinnen.

T. Hitomi et al. Nippon Dojo Hiryogaku Zasshi, 1965, 36(3), 63 berichten über Untersuchungen zur Hygroskopizität von Kunstdüngern. Diese haben ergeben, dass die Magnesiumsulfat-Harnstoff-Verbindung MgSO₄ • 5-6 CO(NH₂)₂ • 2 H₂O außerordentlich hygroskopisch ist. Daher ist es nicht möglich, diese Verbindungen in festen Düngemitteln einzusetzen.

Der Erfindung liegt daher die Aufgabe zu Grunde, eine nicht zerfließende Zusammensetzung aus Magnesiumsulfat-Harnstoff bereitzustellen, welche die obengenannten Nachteile der Magnesium-Schwefel-Stickstoff-Verbindungen gemäß dem Stand der Technik behebt.

Überraschenderweise wurde gefunden, dass mittels geeigneter Verfahren auf schnelle und einfache Weise die Magnesiumsulfat-Harnstoff-Verbindung der Formel I in sehr guten Ausbeuten als reine, kristalline und lagerstabile Zusammensetzung hergestellt werden kann, die die Nachteile des Standes der Technik nicht aufweist und die insbesondere nicht hygroskopisch ist. Die so erhaltene Zusammensetzung lässt sich daher besonders gut in Düngemittelzusammensetzungen einarbeiten.

Gegenstand der Erfindung ist eine Zusammensetzung, enthaltend wenigstens 80 Gew.-%, insbesondere wenigstens 90 Gew.-%, bezogen auf das Gesamtgewicht der Zusammensetzung, einer Magnesiumsulfat-Harnstoff Verbindung der Formel (I):

[MgSO₄ • m CO(NH₂)₂ • n H₂O] (I),

worin m im Bereich von 0,9 bis 1,1 und n im Bereich von 2,1 bis 3,1 liegen, wobei die Zusammensetzung, bezogen auf das Gesamtgewicht der Zusammensetzung, weniger als 10 Gew.-%, insbesondere weniger als 5 Gew.-%, freies MgSO₄ in Form des Anhydrates oder in Form von harnstofffreien Hydraten des Magnesiumsulfats wie Bittersalz oder Magnesiumsulfat-Hexahydrat und/oder weniger als 10 Gew.-%, insbebesondere weniger als 5 Gew.-% ungebundenen Harnstoff enthält. Insbesondere weist m in der Formel (I) den Wert 1 auf Insbesondere weist n in der Formel (I) den Wert 3 auf

Die erfindungsgemäßen Zusammensetzungen sind feste, in der Regel kristalline, nicht oder nur gering hygroskopische Zusammensetzungen. Sie weisen, anders als die aus dem Stand der Technik bekannten Zusammensetzungen der Verbindung der Formel I, nur einen geringen Anteil an Edukten wie Harnstoff oder freies Magnesiumsulfat auf. Zudem ist die Verbindung der Formel I in sehr guten Ausbeuten zugänglich und in lagerstabiler Form erhältlich. Außerdem lassen sich diese Zusammensetzungen leicht in Pulverform oder in Form von Granulaten herstellen und daher in einfacher Weise als Düngemittel verwenden oder in konventionelle Düngemittelzusammensetzungen einbringen. Ferner können durch Auflösen der Zusammensetzungen in einem Lösungsmittel, beispielsweise Wasser, Lösungen der Zusammensetzungen hergestellt werden.

In der Regel enthält die erfindungsgemäße Zusammensetzung, weniger als 10 Gew.-%, insbesondere weniger als bezogen auf das Gesamtgewicht der Zusammensetzung, ungebundenen Harnstoff und gleichzeitig weniger als 10 Gew.-%, bezogen auf das Gesamtgewicht der Zusammensetzung, freies MgSO₄ in Form des Anhydrates oder in Form von harnstofffreien Hydraten des Magnesiumsulfats. Vorzugsweise enthält die Zusammensetzung weniger als 5 Gew.-% freies MgSO₄ in Form des Anhydrates oder in Form von harnstofffreien Hydraten des Magnesiumsulfats oder weniger als 5 Gew.-% ungebundenen Harnstoff, jeweils bezogen auf das Gesamtgewicht der Zusammensetzung. Insbesondere enthält die Zusammensetzung weniger als 5 Gew.-%, speziell weniger als 3 Gew.-% freies MgSO₄ in Form des Anhydrates oder in Form von harnstofffreien Hydraten des Magnesiumsulfats und gleichzeitig weniger als 5 Gew.-%, insbesondere weniger als 3 Gew.-% ungebundenen Harnstoff, jeweils bezogen auf das Gesamtgewicht der Zusammensetzung.

Der Anteil an freiem MgSO₄ in Form des Anhydrats oder in Form von Hydraten des Magnesiumsulfats wie Bittersalz, Magnesiumsulfat-Monohydrat oder Magnesiumsulfat-Hexahydrat sowie der Anteil an freiem Harnstoff kann mittels Röntgenpulver-Diffraktometrie durch Vergleich eines Pulverdiffraktogramms mit Referenz-Pulverdiffraktogrammen der Verunreinigungen wie MgSO₄ in Form des Anhydrates, Bittersalz (MgSO₄ • 7 H₂O) oder auch Magnesiumsulfat-Hexahydrat und Harnstoff ermittelt werden. Derartige Methoden sind dem Fachmann bekannt und können in an sich bekannter Weise, beispielsweise mit Hilfe der Pulverröntgen-Diffraktometrie Software: "EVA" Ver. 12.0.0.0 der Fa. Bruker AXS, Datenbank: Powder Diffraction Files (PDF-2, Release 1999; Data Sets 1-49, plus 70-86) des International Center for Diffraction Data (ICDD) durchgeführt werden.

Aus der Abwesenheit der für die Verunreinigungen charakteristischen Reflexe kann zudem geschlossen werden, dass der Anteil der jeweiligen Verunreinigung gemäß qualitativer Auswertung der RDA Diagramme verschwindend gering ist. Die charakteristischen Reflexe für MgSO₄ in Form des Anhydrats, Bittersalz, Magnesiumsulfat-Hexahydrat sowie Harnstoff können der Literatur oder einschlägigen Datenbanken wie denen des International Centre for Diffraction Data (JCPDS) entnommen werden.

Die in den erfindungsgemäßen Zusammensetzungen enthaltene Verbindung der Formel I weist in einem bei 25 °C aufgenommenen Röntgenpulverdiffraktogramm (Cu-K_{α}-Strahlung: λ = 1.5413 Å) wenigstens 3 und insbesondere wenigstens 5 und speziell wenigstens 7 oder alle d-Werte der folgenden der Tabelle 1 auf, wobei sie vorzugsweise wenigstens 3, insbesondere wenigsten 5 und speziell wenigstens 7 der jeweiligen Reflexe aufweist, deren relative Intensität größer 8 %, bezogen auf die Intensität des stärksten Peaks (100 % rel. Intensität) ist. In Tabelle 1 sind die charakteristischen Reflexe der Verbindung I als Netzbenennungsabstände d (in Ängström) angegeben, die sich aus der Bragg'schen Beziehung 20-Werten berechnen lassen.

**Tabelle 1**

| **d-Wert (Å)** | **rel. Intensität (%)** |
|---|---|
| 10,1656 | 100 |
| 3,3994 | 63 |
| 3,8527 | 39 |
| 4,0752 | 21 |
| 2,9318 | 15 |
| 2,7558 | 15 |
| 4,2520 | 13 |
| 3,0064 | 13 |
| 4,8050 | 9 |
| 3,2015 | 9 |
| 5,0928 | 8 |
| 2,2609 | 8 |
| 2,4920 | 8 |
| 6,1384 | 7 |
| 3,5177 | 7 |
| 2,5505 | 7 |
| 2,2649 | 6 |
| 2,9572 | 6 |
| 2,2944 | 6 |
| 2,1393 | 6 |
| 2,1293 | 6 |
| 2,4767 | 5 |

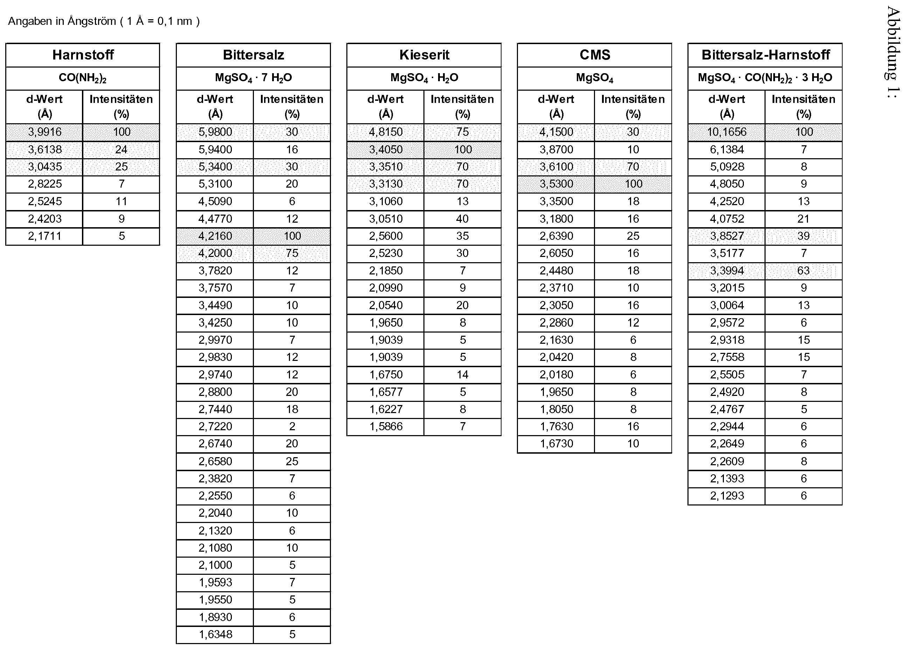

Die in den erfindungsgemäßen Zusammensetzungen enthaltenen Verbindungen können in einem bei 25 °C (Cu-K_{α}-Strahlung: λ = 1.5413 Å) aufgenommenen Röntgenpulverdiffraktogramm der Zusammensetzung anhand der in der folgenden Tabelle 2 angegebene Reflexe identifiziert werden, wobei zur Identifikation typischerweise wenigstens 3 und insbesondere wenigstens 5 und speziell wenigstens 7 der angegebenen d-Werte mit einer relativen Intensität größer 10 % herangezogen werden. Eine vollständige Auflistung der d-Werte ist in Abbildung 1 dargestellt.

**Tabelle 2**

| **Verunreinigung** | **d-Wert (Å)** | **rel. Intensität (%)** |
|---|---|---|
| Harnstoff | 3,9916 | 100 |
| | 3,6138 | 24 |
| | 3,0435 | 25 |
| MgSO₄ · 7 H₂O | 4,2160 | 100 |
| | 4,2000 | 75 |
| | 5,3400 | 30 |
| | 5,9800 | 30 |
| MgSO₄ · H₂O | 3,4050 | 100 |
| | 4,8150 | 75 |
| | 3,3510 | 70 |
| | 3,3130 | 70 |
| MgSO₄ | 3,5300 | 100 |
| | 3,6100 | 70 |
| | 4,1500 | 30 |

Die Stöchiometrie der Verbindung kann durch Elementaranalyse unter Berücksichtigung der vorhandenen Verunreinigungen bestimmt werden.

Es wurde weiterhin ein Verfahren zur Herstellung der erfindungsgemäßen Zusammensetzungen der Magnesiumsulfat-Harnstoff-Verbindung der Formel (I) gefunden, das dadurch gekennzeichnet ist, dass man wasserfreies Magnesiumsulfat mit Harnstoff und Wasser umsetzt. Dieses Verfahren wird im Folgenden auch als Verfahren 1 bezeichnet. Anders als das aus dem Stand der Technik bekannte Verfahren, bei dem Bittersalz mit Harnstoff umgesetzt wird, liefert das erfindungsgemäße Verfahren 1 Zusammensetzungen, welche wenigstens 80 Gew.-%, insbesondere wenigstens 90 Gew.-% der Verbindung der Formel I in hoher Reinheit und weitgehender oder vollständiger Abwesenheit von freiem, d.h. ungebundenen Harnstoff und freiem, d.h. ungebundenem Magnesiumsulfat in Form des Anhydrates oder in Form von Hydraten des Magnesiumsulfats.

In der Regel setzt man im Verfahren 1 wasserfreies Magnesiumsulfat, Harnstoff und Wasser in der für die Verbindung erforderlichen Stöchiometrie ein, d. h. in einem Molverhältnis von Magnesiumsulfat zu Harnstoff im Bereich von 1 : 0,9 bis 1 : 1,1, insbesondere 1 : 0,95 bis 1 : 1,05 und speziell 1 : 0,98 bis 1 : 1,02 und einem Verhältnis von Magnesiumsulfat zu Wasser im Bereich von 1 : 2,9 bis 1: 3,1, insbesondere 1 : 2,95 bis 1 : 3,05 und speziell 1 : 2,98 bis 1 : 3,02.

Das Verfahren 1 umfasst das Vermischen von wasserfreiem Magnesiumsulfat, Harnstoff und Wasser. Insbesondere wird man zur Umsetzung gemäß Verfahren 1 wasserfreies Magnesiumsulfat mit Harnstoff im obengenannten Molverhältnis vermischen und hierzu Wasser im obengenannten Molverhältnis geben. Eine mögliche Ausgestaltung des Verfahrens 1 wird in Beispiel 1 beschrieben.

Die Umsetzung gemäß Verfahren 1 ist exotherm. Hierbei hat es sich als vorteilhaft erwiesen, die Umsetzung der Reaktionspartner wasserfreies Magnesiumsulfat, Harnstoff und Wasser im Verfahren 1 unter Ausnutzung der Reaktionswärme durchzuführen. Vorzugsweise wird man dabei eine Temperatur von 70 °C, insbesondere 60°C nicht überschreiten. Gegebenenfalls wird man daher das Reaktionsgemisch kühlen. Insbesondere erfolgt die Umsetzung bei Temperaturen im Bereich von 30 bis 60 °C. Gegebenenfalls wird man Maßnahmen treffen, um Wasser, welches während der Umsetzung verdampft, in die Reaktion zurückzuführen. Vorzugsweise wird man während der Umsetzung die in der Regel flüssige oder plastische Reaktionsmischung durchmischen, beispielsweise mittels geeigneter Rühr- oder Knetvorrichtungen oder mittels Extruder.

Nach Beendigung der Reaktion lässt man die entstandene Zusammensetzung in der Regel auf Umgebungstemperatur abkühlen. Hierbei erhält man typischerweise eine feste Masse, die in an sich bekannter Weise zerkleinert werden kann.

Gemäß einer bevorzugten Ausführungsform des Verfahrens 1 wird das flüssige oder plastische Reaktionsgemisch während der Umsetzung oder unmittelbar im Anschluss an die Umsetzung, wenn die Reaktionsmasse noch plastisch ist, gezielt in eine gewünschte Partikelgröße und - form überführt, z. B. durch Granulierung oder Zerkleinerung. Das Granulieren kann in an sich üblicher Weise erfolgen, z. B. indem man die Umsetzung in einem Mischer oder Rolltrommel oder Granulierteller durchführt. Üblicherweise wird man die Granulierung oder Zerkleinerung in einer Weise durchführen, dass im erhaltenen Granulat maximal 10 Gew.-% der Partikel einen Durchmesser oberhalb 5 mm aufweisen. Vorzugsweise wird man das Granulieren so durchführen, dass wenigstens 90 Gew.-% der Partikel einen Teilchendurchmesser im Bereich von 0,5 bis 5 mm aufweisen. Der Teilchendurchmesser kann durch Siebanalyse bestimmt werden.

Es ist aber auch möglich, die nach dem Abkühlen des Reaktionsgemischs erhaltene feste Zusammensetzung zu zerkleinern. Für die Zerkleinerung der Zusammensetzung können übliche Vorrichtungen zum Zerkleinern von Feststoffen eingesetzt werden, vorzugsweise Schlag- oder Prallmühlen, unter anderem auch Backenbrecher, Rundbrecher, Walzenbrecher, Hammerbrecher, Daumenbrecher, Schneckenbrecher, Wälzmühlen, Schlag- und Schleudermühlen. Üblicherweise wird man die Zerkleinerung so lange durchführen, bis mindestens 90 Gew.-% der Partikel einen Durchmesser kleiner 5 mm aufweisen. Vorzugsweise wird man das Zerkleinern so durchführen, dass wenigstens 90 Gew.-% der Partikel einen Teilchendurchmesser im Bereich von 0,5 bis 5 mm aufweisen. Der Teilchendurchmesser kann durch Siebanalyse bestimmt werden.

Das erfindungsgemäße Verfahren 1 und das weiter unten beschriebene Verfahren 3 ermöglichen es, die Verbindung der Formel I in hoher Ausbeute, in der Regel praktisch zu 100 %, bezogen auf Magnesiumsulfat und Harnstoff herzustellen. Der Anteil an freiem Magnesiumsulfat, sei es in Form des Anhydrats oder eines harnstofffreien Hydrates in der erhaltenen Zusammensetzung beträgt weniger als 10 Gew.-% und insbesondere weniger als 5 Gew.-%. Des Weiteren ermöglicht das erfindungsgemäße Verfahren Zusammensetzungen der Verbindung der Formel I, die weniger als 10 Gew.-% und insbesondere weniger als 5 Gew.-% an ungebundenem Harnstoff enthalten, herzustellen.

Anders als im Verfahren des Standes der Technik erfordern die erfindungsgemäßen Verfahren 1 und 3 keine Filtrations- und Trocknungsschritte und sind daher besonders einfach und mit hoher Energie-Effizienz durchführbar.

Neben dem zuvor beschriebenen Verfahren 1 wurde weiterhin ein Verfahren 2 zur Herstellung von Magnesiumsulfat-Harnstoff-Verbindungen der Formel (I) gefunden, das dadurch gekennzeichnet ist, dass man zunächst eine Mischung Bittersalz (MgSO₄ · 7 H₂O) mit Harnstoff im Molverhältnis Bittersalz zu Harnstoff im Bereich von 1 : 0,9 bis 1 : 1,1, insbesondere 1 : 0,95 bis 1 : 1,05 und speziell 1 : 0,98 bis 1 : 1,02 umsetzt und zu der erhaltenen Mischung im Verlauf der Umsetzung weiteren Harnstoff zugibt. Dieses Verfahren wird im Folgenden als Verfahren 2 bezeichnet. Eine mögliche Ausgestaltung des Verfahrens 2 wird in Beispiel 2 beschrieben.

Vorzugsweise gibt man im Verlauf der Umsetzung gemäß Verfahren 2 weiteren Harnstoff in einer Menge von 10 bis 30 mol-%, bezogen auf die zunächst eingesetzte Harnstoffmenge, zu dem Gemisch aus Bittersalz (MgSO₄ • 7 H₂O) und Harnstoff. Die Zugabe kann in einer Portion oder in mehreren Portionen oder auch kontinuierlich erfolgen. Die Zugabe des weiteren Harnstoffs erfolgt in der Regel zu einem Zeitpunkt, an dem Bittersalz (MgSO₄ • 7 H₂O) und Harnstoff bereits miteinander vermischt wurden.

Vorzugsweise erfolgt die Umsetzung gemäß Verfahren 2 bei Temperaturen im Bereich von 20 bis 70 °C und insbesondere im Bereich von 40 bis 60 °C. Da die Umsetzung gemäß Verfahren 2 kaum Wärmetönung aufweist, wird man gegebenenfalls das Gemisch aus Bittersalz und Harnstoff auf die gewünschte Temperatur erwärmen.

Vorzugsweise wird man während der Umsetzung die in der Regel flüssige oder plastische Reaktionsmischung durchmischen, beispielsweise mittels geeigneter Rühr- oder Knetvorrichtungen.

Anders als im Verfahren 1 fällt das im Verfahren 2 erhaltene Reaktionsgemisch in Form einer wässrigen Suspension an. Hieraus kann die erfindungsgemäße Zusammensetzung durch übliche Methoden der Trennung von festen und flüssigen Phasen isoliert werden, beispielsweise durch Filtration oder Zentrifugation. In der Regel wird man einen Trocknungsschritt anschließen. Vorzugsweise erfolgt die Trocknung bei Temperaturen unterhalb 60°C, vorzugsweise im Bereich von 30 bis < 60 °C und insbesondere im Bereich von 30 bis 50 °C.

Die nach Verfahren 2 erhältliche erfindungsgemäße Zusammensetzung fällt typischerweise in feinteiliger Form an, worin weniger als 90 Gew.-% der Partikel einen Durchmesser oberhalb 1 mm aufweisen. Vorzugsweise weisen wenigstens 90 Gew.-% der Partikel einen Teilchendurchmesser kleiner 2 mm auf Der Teilchendurchmesser kann durch Siebanalyse bestimmt werden.

Das erfindungsgemäße Verfahren 2 ermöglicht es ebenfalls, die Verbindung der Formel I in hoher Ausbeute, in der Regel wenigstens 70 %, bezogen auf Magnesiumsulfat, herzustellen. Der Anteil an freiem Magnesiumsulfat, sei es in Form des Anhydrats oder eines harnstofffreien Hydrates in der erhaltenen Zusammensetzung beträgt weniger als 10 Gew.-% und insbesondere weniger als 5 Gew.-%. Des Weiteren ermöglicht das erfindungsgemäße Verfahren, Zusammensetzungen der Verbindung der Formel I, die weniger als 10 Gew.-% und insbesondere weniger als 5 Gew.-% an ungebundenen Harnstoff enthalten, herzustellen.

Neben dem zuvor beschriebenen Verfahren 1 und 2 wurde weiterhin ein Verfahren 3 zur Herstellung von Magnesiumsulfat-Harnstoff-Verbindungen der Formel (I) gefunden, das dadurch gekennzeichnet ist, dass man zunächst eine Mischung von wasserfreiem Magnesiumsulfat, Harnstoff und Bittersalz (MgSO₄ · 7 H₂O) umsetzt. Eine mögliche Ausgestaltung des Verfahrens 3 wird in Beispiel 3 beschrieben.

Vorzugsweise setzt man im Verfahren 3 wasserfreies Magnesiumsulfat, Harnstoff und Bittersalz in der für die Verbindung erforderlichen Stöchiometrie ein, d. h. in einem Molverhältnis wasserfreies Magnesiumsulfat: Harnstoff im Bereich von 1 : 1,65 bis 1 : 1,85 und das Molverhältnis wasserfreies Magnesiumsulfat: Bittersalz im Bereich von 1 : 0,65 bis 1 : 0,85, wobei das Molverhältnis von Harnstoff zur Gesamtmenge an wasserfreiem Magnesiumsulfat und Bittersalz im Bereich von 0,9 : 1 bis 1,1 : 1 liegt. Dieses Verfahren wird im Folgenden als Verfahren 3 bezeichnet.

Vorzugsweise wird zunächst die Mischung der obengenannten Edukte auf 30 bis 60 °C, vorzugweise auf etwa 50 °C (d.h. 40 bis 55°C), erwärmt. Vorzugsweise wird man während der Umsetzung die in der Regel flüssige oder plastische Reaktionsmischung durchmischen, beispielsweise mittels geeigneter Rühr- oder Knetvorrichtungen oder mittels Extruder. Die entstehende pastöse Masse der entstandenen Zusammensetzung erstarrt nach Abkühlung auf Umgebungstemperatur. Hierbei erhält man typischerweise eine feste Masse, die in an sich bekannter Weise zerkleinert werden kann.

Gemäß einer bevorzugten Ausführungsform des Verfahrens 3 wird das flüssige oder plastische Reaktionsgemisch während der Umsetzung oder unmittelbar im Anschluss an die Umsetzung, wenn die Reaktionsmasse noch plastisch ist, gezielt in eine gewünschte Partikelgröße und - form überführt, z. B. durch Granulierung oder Zerkleinerung. Das Granulieren kann in an sich üblicher Weise erfolgen, z. B. indem man die Umsetzung in einem Mischer oder Rolltrommel oder Granulierteller durchführt. Üblicherweise wird man die Granulierung oder Zerkleinerung in einer Weise durchführen, dass im erhaltenen Granulat maximal 10 Gew.-% der Partikel einen Durchmesser oberhalb 5 mm aufweisen. Vorzugsweise wird man das Granulieren so durchführen, dass wenigstens 90 Gew.-% der Partikel einen Teilchendurchmesser im Bereich von 0,5 bis 5 mm aufweisen. Der Teilchendurchmesser kann durch Siebanalyse bestimmt werden.

Es ist aber auch möglich, die nach dem Abkühlen des Reaktionsgemischs erhaltene feste Zusammensetzung zu zerkleinern. Für die Zerkleinerung der Zusammensetzung können übliche Vorrichtungen zum Zerkleinern von Feststoffen eingesetzt werden, vorzugsweise Schlag- oder Prallmühlen, unter anderem auch Backenbrecher, Rundbrecher, Walzenbrecher, Hammerbrecher, Daumenbrecher, Schneckenbrecher, Wälzmühlen, Schlag- und Schleudermühlen. Üblicherweise wird man die Zerkleinerung so lange durchführen, bis mindestens 90 Gew.-% der Partikel einen Durchmesser kleiner 5 mm aufweisen. Vorzugsweise wird man das Zerkleinern so durchführen, dass wenigstens 90 Gew.-% der Partikel einen Teilchendurchmesser im Bereich von 0,5 bis 5 mm aufweisen. Der Teilchendurchmesser kann durch Siebanalyse bestimmt werden.

Anders als im Verfahren des Standes der Technik erfordert das erfindungsgemäße Verfahren 3 keine Filtrations- und Trocknungsschritte und ist daher besonders einfach und mit hoher Energie-Effizienz durchführbar. Außerdem muss bei Verfahren 3 kein zusätzliches Wasser in die Reaktionsmischung gegeben werden.

Die Erfindung betrifft weiterhin die Verwendung der erfindungsgemäßen Zusammensetzungen der Formel (I) als Düngemittel oder als Düngemitteladditiv.

Die erfindungsgemäße Zusammensetzung ist als solche als Düngemittel geeignet und stellt für sich eine Stickstoff-Magnesium-Schwefel Düngemittelzusammensetzung dar. Sie eignet sich jedoch insbesondere auch als Stickstofflieferant für konventionelle Magnesium-Schwefel-Düngemittelzusammensetzungen. Durch Additivierung konventioneller Magnesium-Schwefel-Düngemittelzusammensetzungen können somit in einfacher Weise Stickstoff-Magnesium-Schwefel-Düngemittelzusammensetzungen bereitgestellt werden.

Derartige Stickstoff-Magnesium-Schwefel-Düngemittelzusammensetzungen enthalten die Verbindung der Formel I typischerweise in einer Menge von 1 bis 100 Gew.-%.

Neben der Verbindung der Formel I und gegebenenfalls Magnesiumsulfat, vorzugsweise in Form von Bittersalz, können die Stickstoff-Magnesium-Schwefel-Düngemittelzusammensetzungen weitere Makronährstoffe wie Phosphor, vorzugsweise in Form von Phosphaten, und/oder Kalium, sowie gegebenenfalls Mikronährstoffe wie Mangan, Zink, Kupfer, Eisen, Kobalt, Molybdän und/oder Bor enthalten. Mangan und Zink werden dabei vorzugsweise in Form ihrer Sulfate eingesetzt. Kupfer, Kobalt und Eisen werden vorzugsweise in Form von Chelaten, z. B. mit EDTA, eingesetzt. Bor wird vorzugsweise als Natriumborat oder Borsäure eingesetzt. Molybdän wird vorzugsweise als Natrium- oder Ammoniummolybdat oder als Mischung davon eingesetzt.

Vorzugsweise wird die Zusammensetzung als Additiv in Bittersalz enthaltenden Düngemitteln verwendet. Derartige Düngemittel bzw. Düngemittelzusammensetzungen sind neu und ebenfalls Gegenstand der Erfindung. Insbesondere enthält die erfindungsgemäße Düngemittelzusammensetzung neben Bittersalz 1 bis 90 Gew.-%, insbesondere 5 bis 50 Gew.-% und speziell 10 bis 30 Gew.-%, bezogen auf das Gesamtgewicht der Düngemittelzusammensetzung, der Verbindung der Formel I sowie gegebenenfalls ein oder mehrere der vorgenannten Mikronährstoffe. Der Anteil der Mikronährstoffe, gerechnet als Element, wird in der Regel nicht mehr als 30 Gew.-%, bezogen auf das Gesamtgewicht der Düngemittelzusammensetzung, betragen und liegt, sofern erwünscht, häufig im Bereich von 0,1 bis 10 Gew.-%.

Die erfindungsgemäße Zusammensetzung der Verbindung der Formel I kann auch gemeinsam mit sogenannten Nitrifikationsinhibitoren und/oder Ureaseinhibitoren verwendet werden. Dabei kann die erfindungsgemäße Zusammensetzung der Verbindung der Formel I entweder im Gemisch mit Nitrifikationsinhibitoren oder im Gemisch mit Ureaseinhibitoren oder im Gemisch Nitrifikationsinhibitoren und Ureaseinhibitoren verwendet werden.

Düngemittelzusammensetzungen, die neben wenigstens einer Verbindung der Formel I in Form der erfindungsgemäßen Zusammensetzung und wenigstens einen weiteren Bestandteil enthalten, der unter Ureaseinhibitoren und Nitrifikationsinhibitoren ausgewählt ist, sind ebenfalls Gegenstand der vorliegenden Erfindung.

Geeignete Ureaseinhibitoren sind dem Fachmann bekannt, beispielsweise aus Kiss et al. (Kiss, S., Simihäian, M. 2002, Improving Efficiency of Urea Fertilizers by Inhibition of Soil Urease Activity, ISBN 1-4020-0493-1, Kluwer Academic Publishers, Dordrecht, The Netherland). Geeignete Ureaseinhibitioren sind vor allem N-Alkylphosphorsäuretriamide und N-Alkylthiophosphorsäuretriamide und deren Gemischen, wie sie z. B. aus WO 2009/079994 und der dort zitierten Literatur bekannt sind. Bevorzugt sind N-n-Butylthiophosphorsäuretriamid (NBPT), N-n-Propylthiophosphorsäuretriamid (NPPT) und deren Gemische.

Geeignete Nitrifikationsinhibitoren sind neben Dicyandiamid vor allem Pyrazole und deren Säureadditionssalzen, insbesondere deren Phosphorsäureadditionssalze, sowie 1-Carboxyalkylpyrazole und deren Gemische. Hierbei können die Pyrazole und 1-Carboxyalkylpyrazole an den Kohlenstoffatomen durch ein oder mehrere, z. B. 1 oder 2 Substituenten aus der Gruppe C₁-C₄-Alkyl, insbesondere Methyl, Nitro und Halogen, insbesondere Chlor, substituiert sein. Derartige Verbindungen und ihre Verwendung als Nitrifikationsinhibitoren sind beispielsweise aus der US 3635690, der US 4969946, der EP 0808298 und der EP 1120388. Bevorzugte Nitrifikationsinhibitoren sind 3-Methylpyrazolverbindungen wie 4-Chlor-3-methylpyrazol und dessen Säureadditionssalze, N-Hydroxymethyl-4-chlor-3-methylpyrazol und dessen Säureadditionssalze, sowie 3,4-Dimethylpyrazol (DMP)-Verbindungen wie 2-(3,4-Dimethylpyrazol-1-yl)-bernsteinsäure, N-Hydroxymethyl-3,4-dimethylpyrazol und dessen Säureadditionssalze sowie vor allem 3,4-Dimethylpyrazol und die Säureadditionssalze des 3,4-Dimethylpyrazols, speziell seine Phosphorsäureadditionssalze (DMPP).

Derartige Düngemittelzusammensetzungen enthalten die Verbindung der Formel I und den wenigstens einen weiteren Bestandteil aus der Gruppe der Nitrifikationsinhibitoren und Ureaseinhibitoren in der Regel in einer Menge von 0,001 bis 5 Gew.-%, insbesondere in einer Menge von 0,002 bis 3 Gew.-%, bezogen auf das Gesamtgewicht der Düngemittelzusammensetzung.

Sofern derartige Düngemittelzusammensetzungen wenigstens einen Ureaseinhibitor enthalten, beträgt die Konzentration an Ureaseinhibitor in der Regel 0,001 bis 3 Gew.-%, insbesondere 0,002 bis 2 Gew.-%, bezogen den Harnstoff in der Düngemittelzusammensetzung.

Sofern derartige Düngemittelzusammensetzungen wenigstens einen Nitrifikationsinhibitor enthalten, beträgt die Konzentration an Nitrifikationsinhibitor in der Regel 0,01 bis 3 Gew.-%, insbesondere 0,02 bis 2 Gew.-%, bezogen auf das Gesamtgewicht der Düngemittelzusammensetzung, im Falle von Säureadditionssalzen von Pyrazolverbindungen, gerechnet als Salz.

Sofern derartige Düngemittelzusammensetzungen wenigstens einen Ureaseinhibitor und wenigstens einen Nitrifikationsinhibitor enthalten, beträgt die Gesamtkonzentration an Nitrifikationsinhibitor + Ureaseinhibitor in der Regel 0,011 bis 5 Gew.-%, insbesondere 0,022 bis 3 Gew.-%, bezogen auf das Gesamtgewicht der Düngemittelzusammensetzung. Typischerweise beträgt dann das Gewichtsverhältnis von dem wenigstens einen Nitrifikationsinhibitor zu dem wenigstens einen Ureaseinhibitor in der Regel 1 : 10 bis 10 : 1 und vorzugsweise 1 : 5 bis 5 : 1.

Düngemittelzusammensetzungen, die wenigstens neben wenigstens einer Verbindung der Formel I in Form der erfindungsgemäßen Zusammensetzung und wenigstens einen weiteren Bestandteil enthalten, der unter Ureaseinhibitoren und Nitrifikationsinhibitoren ausgewählt ist, können außerdem gegebenenfalls Magnesiumsulfat, vorzugsweise in Form von Bittersalz, und/oder weitere Makronährstoffe wie Phosphor, vorzugsweise in Form von Phosphaten, und/oder Kalium, sowie gegebenenfalls Mikronährstoffe wie Mangan, Zink, Kupfer, Eisen, Kobalt, Molybdän und/oder Bor enthalten. Mangan und Zink werden dabei vorzugsweise in Form ihrer Sulfate eingesetzt. Kupfer, Kobalt und Eisen werden vorzugsweise in Form von Chelaten, z. B. mit EDTA, eingesetzt. Bor wird vorzugsweise als Natriumborat oder Borsäure eingesetzt. Molybdän wird vorzugsweise als Natrium- oder Ammoniummolybdat oder als Mischung davon eingesetzt. Bezüglich der Mengenanteile der Verbindung der Formel I und den Mikronährstoffen gilt das zuvor Gesagte.

Wie schon oben erwähnt, können durch Auflösen der erfindungsgemäßen Zusammensetzungen in einem Lösungsmittel, beispielsweise Wasser, Lösungen der erfindungsgemäßen Zusammensetzungen hergestellt werden. Auf diese Weise können die erfindungsgemäßen Zusammensetzungen unter anderem in Flüssigdüngern zur Bewässerungsdüngung (Fertigation) verwendet werden. Wasser, das von den Wurzeln der Pflanzenkulturen nicht aufgenommen kann, da es z. B. im Substrat oder in für Wurzel nicht erreichbare Bodenschichten versickert, wird als Überschusswasser bezeichnet. Die Durchführung der künstlichen Bewässerungsdüngung mit Flüssigdüngung der erfindungsgemäßen Zusammensetzung kann so erfolgen, dass im Wesentlichen kein Überschusswasser anfällt. Bevorzugt wird die Bewässerungsdüngung mit Flüssigdünger der erfindungsgemäßen Zusammensetzung, insbesondere ein Gemisch aus Flüssigdünger der erfindungsgemäßen Zusammensetzung mit weiteren Additiven, z. B. UreaseInhibitoren, so durchgeführt, dass kein Überschusswasser anfällt.

Die erfindungsgemäße Zusammensetzung und die erfindungsgemäßen Verfahren werden durch die nachfolgenden Beispiele und der Abbildung 1 näher erläutert.

Abbildung 1: Zusammenstellung der charakteristischen Reflexe (als d-Werte in Ängström angegeben) und deren relative Intensität im Röntgenpulverdiffraktogramm von Harnstoff (CO(NH₂)₂), Bittersalz (MgSO₄ · 7 H₂O), Kieserit (MgSO₄ · H20), wasserfreies Magnesiumsulfat (MgSO₄) und der Verbindung der Formel I (MgSO₄ · CO(NH₂)₂ · 3 H₂O).

Die Aufnahme der Röntgenpulverdiffraktogramme erfolgte mit einem Typ D 8 Advance Diffraktometer der Fa. Bruker, AXS (298 K, Cu-Kα-Strahlung: λ = 1.5413 Å), Schrittweite: 0,018385738, Schrittdauer: 0,2 Sekunden, Detektor: Lynx Eye.

Die Elementaranalysen erfolgten durch:
N-Best. DIN ISO 13878, TOC-Best., DIN EN 1484, Mg / S Verbandsmethode LUFA (K+S 0905.01)

### Beispiel 1

256,5 g Magnesiumsulfat wasserfrei und 128 g geprillter Harnstoff wurden in einem Rührbehälter gut vermischt. Anschließend wurde unter stetigem intensivem Rühren 115,5 g Wasser zugegeben. Es bildete sich zunächst ein dünnflüssiger Brei, und die Mischung erwärmte sich auf 52 °C. Mit weiterer Rührzeit wurde die Mischung immer fester, es bildeten sich Klumpen, die immer wieder zerdrückt wurden. Nach 10 Stunden war die Masse stichfest und nach 24 Stunden sehr hart. Nach weiteren 24 Stunden Ruhezeit wurde die Masse mit dem Hammer auf die gewünschte Korngröße < 0,8 mm zerkleinert. Die Auswage betrug ca. 500 g.

Chemische Analyse des Feststoffes: 51,5 % MgSO₄; 27,1 % Harnstoff; 21,4 % Wasser. Der Anteil an Bittersalz und wasserfreiem Magnesiumsulfat betrug 0 Gew.-%.
Der rechnerische Anteil an freiem Harnstoff betrug 1,5 Gew.-%.
Die theoretische Zusammensetzung der reinen Verbindung MgSO₄ · CO(NH₂)₂ · 3 H₂O ist 51,3% MgSO₄, 25,6 % Harnstoff und 23,1 % Wasser bzw. 11,9 % N, 17,2 % MgO und 13,7 % S.

### Beispiel 2

562,8 g Bittersalz und 137,2 g geprillter Harnstoff wurden 24 Stunden bei 50 °C gerührt. Es entsteht eine dünnflüssige Suspension, in die dann weitere 28 g Harnstoff gegeben wurden. Anschließend wurde weitere 24 Stunden bei 50 °C gerührt. Nach Abkühlen auf 30 °C wurde der Feststoff abfiltriert und der entwässerte, zerkleinerte Filterkuchen 10 Minuten bei 40 °C im Laborfließbett getrocknet. Die Ausbeute an Feststoff betrug 521 g.

Chemische Analyse des getrockneten Feststoffes: 49,1 % MgSO₄; 27,8 % Harnstoff; 23,1 % Wasser
Der Anteil an Bittersalz und wasserfreiem Magnesiumsulfat betrug 0 Gew.-%.
Der rechnerische Anteil an freiem Harnstoff betrug 2,2 Gew.-%.

### Beispiel 3

137,6 g wasserfreies Magnesiumsulfat und 120,1 g geprillter Harnstoff und 211,2 g Bittersalz wurden gut vermischt und auf 50 ° C erwärmt. Innerhalb von 1 Stunde- veränderte sich die Konsistenz. Die Mischung wurde zunächst feucht und klumpig. Beim gelegentlichen Rühren entstand ein Brei, der sich zunehmend verfestigte. Nach Abkühlung auf Raumtemperatur war die Mischung hart und wurde zerkleinert.
Die Auswage betrug ca. 469 g.
Chemische Analyse des Feststoffes: 54,0 % MgSO₄-; 26,5 % Harnstoff; 19,5 % Wasser Der Anteil an Bittersalz und wasserfreiem Magnesiumsulfat betrug 2,7 Gew.-%.
Der Anteil an freiem Harnstoff betrug 0,9 Gew.-%.

### Vergleichsbeispiel 1

80,4 g Bittersalz und 19,6 g geprillter Harnstoff wurden 24 Stunden bei 23 °C gerührt. Es entsteht eine dünnflüssige Suspension. Der Feststoff wurde abfiltriert und der entwässerte, zerkleinerte Filterkuchen 2 h bei 35 °C getrocknet. Man erhielt einen klammen, klebrigen Feststoff in einer Ausbeute von 66,6 g.

Chemische Analyse des getrockneten Feststoffes: 46,6 % MgSO₄; 18,0 % Harnstoff; 35,4 % Wasser. Der rechnerische Anteil an Bittersalz und wasserfreiem Magnesiumsulfat betrug mehr als 20 Gew.-%.

### Vergleichsbeispiel 2

80,4 g Bittersalz und 19,6 g geprillter Harnstoff wurden 24 Stunden bei 50 °C gerührt. Es entsteht eine dünnflüssige Suspension. Der Feststoff wurde abfiltriert und der entwässerte, zerkleinerte Filterkuchen 2 h bei 35 °C getrocknet. Man erhielt einen klammen, klebrigen Feststoff in einer Ausbeute von 56,0 g.

Chemische Analyse des getrockneten Feststoffes: 48,5 % MgSO₄; 23,3 % Harnstoff; 28,2 % Wasser.

## Patentansprüche

1. Zusammensetzung, enthaltend wenigstens 80 Gew.-%, bezogen auf das Gesamtgewicht der Zusammensetzung, einer Magnesiumsulfat-Harnstoff Verbindung der Formel (I):
[MgSO₄ • m CO(NH₂)₂ • n H₂O] (I),
worin m im Bereich von 0,9 bis 1,1 und n im Bereich von 2,9 bis 3,1 liegen, wobei die Zusammensetzung, bezogen auf das Gesamtgewicht der Zusammensetzung, weniger als 10 Gew.-% freies MgSO₄ in Form des Anhydrates oder in Form von Hydraten des Magnesiumsulfats und weniger als 10 Gew.-% ungebundenen Harnstoff.

2. Zusammensetzung nach Anspruch 1, enthaltend, bezogen auf das Gesamtgewicht der Zusammensetzung, weniger als 5 Gew.-% freies MgSO₄ in Form des Anhydrates oder in Form von Hydraten des Magnesiumsulfats und weniger als 5 Gew.-% ungebundenen Harnstoff.

3. Zusammensetzung nach einen der vorherigen Ansprüche, worin m in Formel (I) den Wert 1,0 aufweist.

4. Zusammensetzung nach Anspruch 3, worin n den Wert 3,0 aufweist.

5. Verfahren zur Herstellung einer Zusammensetzung nach einem der vorherigen Ansprüche, **dadurch gekennzeichnet, dass** man wasserfreies Magnesiumsulfat mit Harnstoff und Wasser umsetzt, wobei man zu einer Mischung von wasserfreiem Magnesiumsulfat und Harnstoff Wasser gibt wobei die Umsetzung bei einer Temperatur im Bereich von 30 bis 60 °C erfolgt, wobei während der Umsetzung die Reaktionsmischung durchmischt wird.

6. Verfahren nach Anspruch 5, **dadurch gekennzeichnet, dass** man wasserfreies Magnesiumsulfat, Harnstoff und Wasser in einem Molverhältnis von Magnesiumsulfat zu Harnstoff im Bereich von 1 : 0,9 bis 1 : 1,1 und Magnesiumsulfat zu Wasser im Bereich von 1 : 2,9 bis 1 : 3,1 einsetzt.

7. Verfahren nach einem der Ansprüche 5 oder 6, **dadurch gekennzeichnet, dass** man die bei der Umsetzung entstehende Zusammensetzung während der Umsetzung oder unmittelbar im Anschluss daran, wenn die Reaktionsmasse noch plastisch ist, granuliert.

8. Verfahren zur Herstellung von Zusammensetzungen nach einem der Ansprüche 1 bis 4, **dadurch gekennzeichnet, dass** man zunächst eine Mischung Bittersalz mit Harnstoff im Molverhältnis 1 : 0,9 bis 1 : 1,1 umsetzt und zu der erhaltenen Mischung weiteren Harnstoff zugibt.

9. Verfahren nach Anspruch 8, **dadurch gekennzeichnet, dass** man 10 bis 30 mol-%, bezogen auf die zunächst eingesetzte Harnstoffmenge, weiteren Harnstoff zugibt.

10. Verfahren zur Herstellung von Zusammensetzungen nach einem der Ansprüche 1 bis 4, **dadurch gekennzeichnet, dass** man wasserfreies Magnesiumsulfat, Harnstoff und Bittersalz umsetzt, wobei das Molverhältnis wasserfreies Magnesiumsulfat: Harnstoff im Bereich von 1 : 1,65 bis 1 : 1,85 und das Molverhältnis wasserfreies Magnesiumsulfat: Bittersalz im Bereich von 1 : 0,65 bis 1 : 0,85 liegt.

11. Verwendung einer Zusammensetzung nach einem der Ansprüche 1 bis 4 als Düngemittel oder als Zusatz in Düngemittel.

12. Stickstoff-Magnesium-Schwefel-Düngemittelzusammensetzung, enthaltend eine Zusammensetzung nach einem der Ansprüche 1 bis 4 und Bittersalz.

13. Stickstoff-Magnesium-Schwefel-Düngemittelzusammensetzung, enthaltend eine Zusammensetzung nach einem der Ansprüche 1 bis 4 und wenigstens einen weiteren Bestandteil, der unter Ureaseinhibitoren und Nitrifikationsinhibitoren ausgewählt ist.

14. Verfahren zur Bewässerungsdüngung von landwirtschaftlichen oder gärtnerisch genutzten Substraten, bei dem man Wasser, welches eine Zusammensetzung gemäß einem der Ansprüche 1 bis 4 oder ein Stickstoff-Magnesium-Schwefel-Düngemittel nach einem der Ansprüche 12 und 13 und gegebenenfalls ein oder mehrere weitere Additive so auf ein Substrat einbringt und/oder aufbringt, dass im Wesentlichen kein Überschusswasser anfällt.

## Claims

1. Formulations consisting, at least 80 weight % with respect to the total weight of the formulation, of a magnesium sulphate-urea compound of Formula (I):
**[MgSO₄ • m CO(NH₂)₂** • **n H₂O]** **(I),**
whereby m lies in the range of 0.9 to 1.1 and n in the range of 2.9 to 3.1, whereby the formulation consists of less than 10 weight % of free MgSO₄ in anhydrous form or in the form of hydrates and less than 10 weight % of uncombined urea with respect to the total weight of the formulation.

2. Formulations pursuant to Claim 1, containing less than 5 weight % of free MgSO₄ in anhydrous form or in the form of hydrates of magnesium sulphate and less than 5 weight % of uncombined urea with respect to the total weight of the formulation.

3. Formulations pursuant to either one of the previous claims, whereby m in Formula (I) demonstrates a value of 1.0.

4. Formulations pursuant to Claim 3, whereby n demonstrates a value of 3.0.

5. Processes for manufacturing a formulation pursuant to one of the previous claims, **characterised by** the reaction of anhydrous magnesium sulphate with urea and water, whereby water is added to the mixture of anhydrous magnesium sulphate and urea and whereby the reaction occurs within a temperature range of 30 to 60 °C, whereby the reaction mixture is stirred throughout the period of the reaction.

6. Processes pursuant to Claim 5, **characterised by** the use of anhydrous magnesium sulphate, urea and water in a molar ratio of magnesium sulphate to urea within the range of 1 : 0.9 to 1 : 1.1 and magnesium sulphate to water within the range of 1 : 2.9 to 1 : 3.1.

7. Processes pursuant to either Claim 5 or Claim 6, **characterised by** the granulation of the formulation, which is formed by the reaction, either during the reaction or immediately afterwards, when the reaction mass is still plastic.

8. Processes to manufacturing formulations pursuant to any of the Claims 1 to 4, **characterised by** the initial reaction of a mixture of Epsom salt with urea in the molar ratio of 1 : 0.9 to 1 : 1.1 and the subsequent addition of further urea to the produced mixture.

9. Processes pursuant to Claim 8, **characterised by** the addition of 10 to 30 mol % additional urea with respect to the quantity of urea initially used.

10. Processes to manufacture formulations pursuant to any of the Claims 1 to 4, **characterised by** the reaction of anhydrous magnesium sulphate, urea and Epsom salt, whereby the molar ratio of anhydrous magnesium sulphate : urea is within the range of 1 : 1.65 to 1 : 1.85 and the molar ratio of anhydrous magnesium sulphate : Epsom salt is within the range of 1 : 0.65 to 1 : 0.85.

11. Use of a formulation pursuant to any of the Claims 1 to 4 as fertiliser or as an additive in fertiliser.

12. Nitrogen-magnesium-sulphur fertiliser formulations containing a formulation pursuant to any of the Claims 1 to 4 and Epsom salt.

13. Nitrogen-magnesium-sulphur fertiliser formulations containing a formulation pursuant to any of the Claims 1 to 4 and at least one of the other components, selected from urease inhibitors and nitrification inhibitors.

14. Processes of irrigation fertilisation of agriculturally or horticulturally used substrates, in which water introduces and/or applies, a formulation pursuant to any of the Claims 1 to 4 or a nitrogen-magnesium-sulphur fertiliser pursuant to the Claims 12 and 13 and, if necessary, one or more other additives to a substrate so that, in general, no excess water is formed.

## Revendications

1. Composition, comprenant au moins 80 parties-poids, par rapport au poids total de la composition, d'un composé d'urée et de sulfate de magnésium de la formule (I) :
[MgSO₄ . m CO(NH₂)₂. n H₂O] (I)
pour laquelle m se situe dans la plage de 0,9 à 1,1 et n dans la plage de 2,9 à 3,1, la composition, par rapport au poids total de la composition, contenant moins de 10 parties-poids de MgSO₄ libre sous forme de l'anhydrate ou sous forme de l'hydrate du sulfate de magnésium et moins de 10 parties-poids d'urée non liée.

2. Composition selon la revendication 1, contenant, par rapport au poids total de la composition, moins de 5 parties-poids de MgSO₄ libre sous forme de l'anhydrate ou sous forme de l'hydrate du sulfate de magnésium et moins de 5 parties-poids d'urée non liée.

3. Composition selon l'une des revendications ci-avant, m présentant la valeur de 1,0 dans la formule (I).

4. Composition selon la revendication 3, n présentant la valeur de 3,0.

5. Procédé pour la fabrication d'une composition selon l'une des revendications ci-avant, **caractérisé par le fait que** l'on mélange du sulfate de magnésium anhydre avec de l'urée et de l'eau, en ajoutant de l'eau à un mélange de sulfate de magnésium anhydre et d'urée, la préparation se faisant à une température comprise dans la plage de 30 à 60 °C, le mélange de réaction étant mélangé pendant la préparation.

6. Procédé selon la revendication 5, **caractérisé par le fait que** l'on utilise du sulfate de magnésium anhydre, de l'urée et de l'eau dans un rapport moléculaire du sulfate de magnésium et de l'urée compris dans la plage de 1 : 0,9 à 1 : 1,1 et du sulfate de magnésium et de l'eau dans un rapport compris dans la plage de 1 : 2,9 à 1 : 3,1.

7. Procédé selon l'une des revendications 5 ou 6, **caractérisé par le fait que** la composition obtenue lors de sa réalisation est granulée pendant sa réalisation ou immédiatement après lorsque la masse de réaction est encore plastique.

8. Procédé de fabrication de compositions selon l'une des revendications 1 à 4, **caractérisé par le fait que** l'on réalise tout d'abord un mélange de sel d'Epsom avec de l'urée sous un rapport moléculaire de 1 : 0,9 à 1 : 1,1 et que de l'urée est encore ajoutée au mélange obtenu.

9. Procédé selon la revendication 8, **caractérisé par le fait que** l'on ajoute encore de l'urée à raison de 10 à 30 mol-% par rapport à la quantité d'urée utilisée dans un premier temps.

10. Procédé de fabrication de compositions selon l'une des revendications 1 à 4, **caractérisé par le fait que** l'on réalise du sulfate de magnésium anhydre, de l'urée et du sel d'Epsom, le rapport moléculaire sulfate de magnésium anhydre : urée se trouvant dans la plage de 1 : 1,65 à 1 : 1,85 et le rapport moléculaire sulfate de magnésium anhydre : sel d'Epsom dans la plage de 1 : 0,65 à 1 : 0,85.

11. Utilisation d'une composition selon l'une des revendications 1 à 4 comme engrais ou comme additif pour engrais.

12. Composition d'engrais à l'azote-magnésium-soufre, contenant une composition selon l'une des revendications 1 à 4 et du sel d'Epsom.

13. Composition d'engrais à l'azote-magnésium-soufre, contenant une composition selon l'une des revendications 1 à 4 et au moins un autre composant, choisi parmi les inhibiteurs d'uréase et es inhibiteurs de nitrification.

14. Procédé de fertilisation par arrosage de substrats utilisés dans le secteur agricole ou du jardinage, pour lequel de l'eau, d'une composition selon l'une des revendications 1 à 4 ou un engrais à l'azote-magnésium-soufre selon l'une des revendications 12 et 13 et, le cas échéant, un ou plusieurs autres additifs sont incorporés à un substrat et/ou déposés de manière à ce qu'un excédent d'eau ne se produise essentiellement pas.
